# EUROPÄISCHE PATENTSCHRIFT

(11) **EP 1 362 576 B1**
(45) Veröffentlichungstag und Bekanntmachung des Hinweises auf die Patenterteilung: **14.09.2011**
(21) Anmeldenummer: 03008416.4
(22) Anmeldetag: 11.04.2003
(51) Int. Cl.: A61Q 5/06, A61K 8/73, A61K 8/36, A61K 8/362, A61K 8/365, A61K 8/81

(54) **Aerosolschaum- oder Pumpschaumprodukt zur Haarbehandlung**
Non aerosol and aerosol hair mousse
Mousse de traitement capillaire en distributeur aérosol ou à pompe

(30) Priorität: 15.05.2002 DE 10221449
(43) Veröffentlichungstag der Anmeldung: 19.11.2003
(73) Patentinhaber: Wella GmbH, 65825 Schwalbach am Taunus (DE)
(72) Erfinder: Birkel, Susanne, 64285 Darmstadt (DE); Wendel, Harald, 64372 Ober-Ramstadt (DE); Franzke, Michael, 64380 Rossdorf (DE); Hannich, Manuela, 63329 Egelsbach (DE)

(56) Entgegenhaltungen:
- EP-A- 0 760 235
- EP-A- 0 943 312
- EP-A- 1 075 832
- DE-A- 4 422 593
- US-A- 3 472 840
- US-A- 6 149 898
- US-B1- 6 248 313

## Beschreibung

Gegenstand der Erfindung ist ein Aerosolschaum- oder ein Pumpschaumprodukt zur Haarbehandlung bestehend aus einer verschäumbaren Zusammensetzung mit einem Gehalt an einem kationischen Cellulosederivat, Chitosan, einer Säure zur teilweisen oder vollständigen Neutralisation des Chitosans und gegebenenfalls einem Treibmittel in einem geeigneten Lösungsmittelsystem, wobei die Menge des kationischen Cellulosederivats zur Chitosanmenge in einem bestimmten Verhältnis steht, sowie ggf. mind. einem Tensid (F), mind. einem haarförbenden Stoff und mind. einem nichtionischen filmbildenden Polymeren.

Schäume zur Haarbehandlung sind bekannt und bestehen in der Regel aus haarfestigenden oder haarpflegenden Substanzen, Schaumbildnern sowie einer geeigneten, in der Regel wasserhaltigen Lösungsmittelbasis. Die Zusammensetzungen werden mittels eines Treibgases oder mittels einer mechanisch betriebenen Pumpschaumvorrichtung unmittelbar vor der Anwendung verschäumt. An die Qualität von derartigen Schaumprodukten werden verschiedene Anforderungen gestellt, die grob in zwei Gruppen aufgeteilt werden können. Die eine Gruppe von Qualitätsanforderungen betrifft die Schaumqualität, d.h. die Beschaffenheit des Schaumes. Hierzu gehören etwa die Fein- oder Grobporigkeit, die Kompaktheit, die zeitliche Stabilität des Schaumes, die Einarbeitbarkeit und Verteilbarkeit auf dem Haar etc.. Die zweite Gruppe von Qualitätsanforderungen betrifft die Wirkungen, die der Schaum nach Einarbeitung in das Haar auf dem Haar hervorruft, d.h. die haarpflegenden Eigenschaften wie z.B. der Griff des Haares im feuchten und trockenen Zustand, die Kämmbarkeit, die Festigung, die Belastung des Haares, den Glanz des Haares etc..

Bei der Optimierung von Schaumprodukten besteht das Problem, dass zwar durch Zusatz spezieller Wirkstoffe eine Verbesserung einiger Qualitätsanforderungen, beispielsweise der Schaumqualität, erreicht werden kann, dies aber mit einer Verschlechterung anderer Qualitätsanforderungen, beispielsweise der haarpflegenden oder haarfestigenden Eigenschaften, erkauft wird. Häufig sind in Schaumprodukten kationische Polymere enthalten aufgrund der guten Substantivität zu menschlichem Haar. Aus der WO 99/66888 A1 sind Schaumkonzentrate bekannt mit einem Gehalt an bestimmten Mengen Polyquaternium-4 und Chitosanlactat. Ein Zusatz von kationischen Cellulosederivaten wie z.B. einem Copolymer aus Hydroxyethylcellulose und Diallyldimethylammoniumchlorid (Polyquaternium-4) hat dabei den Nachteil, dass sich die behandelten Haare glitschig anfühlen und eine relativ hohe Belastung aufweisen. Bei Verwendung von neutralisierten Chitosanen (Chitosoniumsalzen) tritt häufig eine Rückstandsproblematik auf, d.h. auf dem behandelten Haar sind nach der Behandlung kleine Mengen sichtbarer Polymerrückstände zu erkennen. Es bestand daher die Aufgabe, die Qualitätsanforderungen von Schaumprodukten zur Haarbehandlung hinsichtlich Schaumqualität und haarpflegenden bzw. haarfestigenden Eigenschaften weiter zu optimieren und die genannten Nachteile zu vermeiden.

Es wurde nun gefunden, dass die Aufgabe durch das nachfolgend beschriebene Schaumprodukt gelöst wird. Dabei wird unter einem Aerosolschaumprodukt ein Produkt verstanden, welches aus einer flüssigen, verschäumbaren Zusammensetzung besteht, welche zusammen mit einem Treibgas in einer druckdichten Verpackung abgefüllt ist, die Verpackung mit einem Schaumkopf versehen ist und die Zusammensetzung unmittelbar vor der Anwendung in Form eines Schaumes ausgebracht werden kann. Unter einem Pumpschaumprodukt wird ein Produkt verstanden, welches aus einer flüssigen, verschäumbaren Zusammensetzung besteht, welche ohne Treibmittel in einer Verpackung abgefüllt ist, die Verpackung mit einer mechanisch betriebenen Vorrichtung zum Verschäumen (Pumpschäumer) versehen ist und die Zusammensetzung unmittelbar vor der Anwendung in Form eines Schaumes ausgebracht werden kann. Gegenstand der Erfindung ist ein Aerosolschaum- oder Pumpschaumprodukt zur Haarbehandlung, gemäß Anspruch 1.

### Kationisches Cellulosederivat (A)

Das kationische Cellulosederivat (A) ist in der erfindungsgemäßen Zusammensetzung vorzugsweise in einer Menge von 0,1 bis 10, besonders bevorzugt von 0,5 bis 5 Gew.% enthalten. Geeignete Cellulosederivate sind solche, welche mindestens eine quaternäre Ammoniumgruppe aufweisen, z.B. ein Copolymer aus Hydroxyethylcellulose und Diallyldimethylammoniumchlorid (Polyquaternium-4) oder dem Reaktionsprodukt aus Hydroxyethylcellulose und einem mit einer Trialkylammoniumgruppe substituiertem Epoxid (Polyquaternium-10), wobei die Alkylgruppen 1 bis 20 C-Atome aufweisen können und Methylgruppen bevorzugt sind. Das Molekulargewicht liegt vorzugsweise zwischen 100.000 und 600.000, besonders bevorzugt zwischen 200.000 und 400.000. Der Stickstoffgehalt beträgt vorzugsweise 0,5 bis 4%, besonders bevorzugt 1,5 bis 3%. Bevorzugtes Cellulosederivat ist Polyquaternium-4, welches unter den Handelsbezeichnungen Celquat® H100 und Celquat® L200 vertrieben wird, von denen das Celquat® L200 besonders bevorzugt ist.

### Chitosan (B)

Das Chitosan (B) ist in der erfindungsgemäßen Zusammensetzung vorzugsweise in einer Menge von 0,1 bis 10, besonders bevorzugt von 0,5 bis 5 Gew.% enthalten. Bei den erfindungsgemäß einzusetzenden Chitosanen handelt es sich um vollständig oder partiell deacetylierte Chitine. Zur Herstellung von Chitosan geht man vorzugsweise von dem in den Schalenresten von Krustentieren enthaltenem Chitin aus, welches als billiger und natürlicher Rohstoff in großen Mengen zur Verfügung steht. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%.

Ein geeignetes Chitosan wird beispielsweise von der Firma Kyowa Oil&Fat, Japan, unter dem Handelsnamen Flonac® vertrieben. Es hat ein Molekulargewicht zwischen 300.000 bis 700.000 und ist zu 70 bis 80% entacetyliert. Ein bevorzugtes Chitosansalz ist Chitosoniumpyrrolidoncarboxylat, welches beispielsweise unter der Bezeichnung Kytamer® PC von der Firma Amerchol, USA, vertrieben wird. Das enthaltene Chitosan hat ein Molekulargewicht zwischen 200.000 bis 300.000 und ist zu 70 bis 85% entacetyliert. Als Chitosane kommen auch Chitosanderivate, insbesondere alkylierte oder hydroxyalkylierte Derivate, beispielsweise Hydroxyethyl-, Hydroxypropyl- oder Hydroxybutylchitosan in Betracht.

### Neutralisationsmittel (C)

Die Chitosane oder Chitosanderivate liegen in vollständig oder partiell neutralisierter Form vor. Der Neutralisationsgrad liegt vorzugsweise bei mindestens 50%, besonders bevorzugt zwischen 70 und 100%, bezogen auf die Anzahl der freien Basengruppen. Als Neutralisationsmittel werden Ameisensäure, Weinsäure, Äpfelsäure, Zitronensäure, Pyrrolidoncarbonsäure und Salzsäure verwendet Pyrrolidoncarbonsäure und Ameisensäure sind besonders bevorzugt .

### Lösungsmittelsystem (D)

Die erfindungsgemäße Zusammensetzung wird in einem rein wässrigen oder in einem wässrig-alkoholischen Medium mit vorzugsweise mindestens 50 Gew.% Wasser und mindestens 5 Gew.% Alkohol konfektioniert. Das Lösungsmittelsystem ist vorzugsweise in einer Menge von 50 bis 98, besonders bevorzugt von 75 bis 95 Gew.% enthalten. Als Alkohole können insbesondere die für kosmetische Zwecke üblicherweise verwendeten niederen Alkohole mit 1 bis 4 C-Atomen, z.B. Ethanol und Isopropanol enthalten sein. Als zusätzliche Co-Solventien können organische Lösungsmittel oder ein Gemisch aus Lösungsmitteln mit einem Siedepunkt unter 400°C in einer Menge von 0,1 bis 15, bevorzugt von 1 bis 10 Gew.% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol.

### Treibmittel (E)

Das Treibmittel (E) ist in dem erfindungsgemäßen Aersolhaarschaum vorzugsweise in einer Menge von 1 bis 20, besonders bevorzugt von 2 bis 10 Gew.% enthalten. Als Treibmittel sind beispielsweise niedere Alkane, z.B. n-Butan, i-Butan, Propan, Butan oder deren Gemische sowie Dimethylether oder Fluorkohlenwasserstoffe wie 1,1-Difluorethan oder Tetrafluorethan sowie ferner bei den in Betracht kommenden Drücken gasförmig vorliegende Treibmittel, z.B. N₂, N₂O und CO₂ sowie Gemische der vorstehend genannten Treibmittel geeignet. Besonders bevorzugt sind C3- bis C4-Kohlenwasserstoffe.

### Tensid (F)

In einer bevorzugten Ausführungsform ist in dem erfindungsgemäßen Schaumprodukt zusätzlich mindestens ein Tensid (F) enthalten. Das Tensid (F) ist vorzugsweise in einer Menge von 0,01 bis 15, besonders bevorzugt von 0,05 bis 10 Gew.% enthalten. Geeignete Tenside haben emulgierende, lösungsvermittelnde, schaumbildende, schaumverstärkende oder haarpflegende Eigenschaften, sind vorzugsweise kationisch oder nichtionisch und haben einem HLB-Wert von maximal 20, vorzugsweise von 5 bis 18. In einer besonders bevorzugten Ausführungsform ist sowohl ein nichtionisches als auch ein kationisches Tensid enthalten.

Bevorzugte nichtionische Tenside sind ethoxylierte Tenside, wobei die Anzahl der Ethylenoxid-Einheiten zwischen 1 bis 1000, bevorzugt zwischen 1 bis 300, besonders bevorzugt zwischen 1 und 15 liegt. Bevorzugt werden Fettsäureglyceridethoxylate, Fettalkoholethoxylate, Fettaminethoxylate, Fettsäurealkanolamidethoxylate und Fettsäureesterethoxylate mit jeweils 1 bis 50 Ethylenoxideinheiten. Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können, sowie Fettalkohole von oxethyliertem Lanolin oder oxethyliertes Lanolin. Auch die ethoxylierten Fettalkohole, die unter der Typenbezeichnung Dehydol® von der Firma Henkel oder unter der Typenbezeichnung Brij® von der Firma ICI Surfactants vertrieben werden, sind für das erfindungsgemäße Haarbehandlungsmittel geeignet. Unter den Fettsäureesterethoxylaten sind vor allem Diglyceridethoxylate zu nennen, z.B. mit 25 Ethylenoxid-Einheiten ethoxyliertes Rizinusöl mit dem INCI-Namen PEG-25 Hydrogenated Castor Oil (Arlatone® G), mit 35 Ethylenoxid-Einheiten ethoxyliertes Rizinusöl mit dem INCI-Namen PEG-35 Castor Oil (Cremophor® El) oder mit 40 Ethylenoxid-Einheiten ethoxyliertes hydriertes Rizinusöl mit dem INCI-Namen PEG-40 Hydrogenated Castor Oil (Cremophor® RH 410). Weitere geeignete nichtionische Tenside sind ethoxylierte Fettsäurezuckerester, insbesondere ethoxylierte Sorbitanfettsäureester, die als Polysorbate bekannt sind, aber auch nicht ethoxylierte Tenside, wie die Fettsäurezuckerester, die von der Firma ICI Surfactants unter dem Handelsnamen Tween® und Arlacel® vertrieben werden sowie die Alkylpolyglycoside, die von der Firma Henkel unter dem Handelsnamen Plantaren® oder Plantacare® oder von der Firma Seppic unter dem Handelsnamen Oramix® vertrieben werden.

Geeignete kationische Tenside sind weisen eine quaternäre Ammoniumgruppe auf und können durch die allgemeine Formel. (I) dargestellt werden,

N⁽⁺⁾R¹R²R³R⁴ X⁽⁻⁾ (I)

wobei R1 bis R4 unabhängig voneinander Alkylgruppen, Arylgruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten und X⁽⁻⁾ ein Anion darstellt, z.B. ein Halogen, Acetat, Phosphat, Nitrat oder Alkylsulfat, vorzugsweise ein Chlorid oder Bromid. Die aliphatischen Gruppen können zusätzlich zu den C-Atomen und den Wasserstoffatomen auch Querverbindungen oder andere Gruppen wie beispielsweise weitere Aminogruppen enthalten. Beispiele für geeignete kationische Tenside sind die Chloride oder Bromide von Alkyldimethylbenzylammoniumsalzen, Alkyltrimethylammoniumsalze, z.B. Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, z.B. Lauryl- oder Cetylpyridiniumchlorid, Alkylamidoethyltrimethylammoniumethersulfate sowie Verbindungen mit kationischem Charakter wie Aminoxide, z.B. Alkylmethylaminoxide oder Alkylaminoethyldimethylaminoxide. Besonders bevorzugt ist Cetyltrimethylammoniumchlorid.

In einer weiteren, bevorzugten Ausführungsform enthält das erfindungsgemäße Produkt zusätzlich mindestens ein filmbildendes nichtionisches Polymer. Das nichtionische Polymer ist vorzugsweise in einer Menge von 0,01 bis 15 Gew.%, besonders bevorzugt von 0,5 bis 10 Gew.% enthalten. Es kann sich um ein synthetisches, natürliches oder um ein modifiziertes natürliches Polymer handeln. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Unter filmbildenden Polymeren werden erfindungsgemäß solche Polymere verstanden, die bei Anwendung in 0,01 bis 5%iger wässriger, alkoholischer oder wässrig-alkoholischer Lösung oder Dispersion in der Lage sind, auf dem Haar einen Polymerfilm abzuscheiden.

Geeignete synthetische, nichtionische filmbildende Polymere sind Homo- oder Copolymere, die aus mindestens einem der folgenden Monomere aufgebaut sind: Vinylpyrrolidon, Vinylcaprolactam, Vinylester (z.B. Vinylacetat), Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids, Copolymere aus Vinylpyrrolidon und Vinylacetat, Terpolymere aus Vinylpyrrolidon, Vinylacetat und Vinylpropionat, Polyacrylamide, Polyvinylalkohole sowie Polyethylenglykol/Polypropylenglykol Copolymere. Besonders bevorzugt sind Polyvinylpyrrolidon und Copolymere von Vinylpyrrolidon und nichtionischen Comonomeren, z.B. Polyvinylpyrrolidon/Vinylacetat Copolymere. Geeignete natürliche filmbildende Polymere sind z.B. hydroxyalkylierte Polysaccharide, insbesondere Hydroxyalkylcellulose oder Hydroxyalkylguar mit vorzugsweise 2 oder 3 C-Atomen in der Alkylgruppe.

Bei einer besonderen Ausführungsform handelt es sich um ein haarfarbveränderndes Produkt, insbesondere einen Farbfestiger. Die Zusammensetzung enthält dann mindestens einen haarfärbenden Stoff. Hierbei kann es sich um lösliche, organische Farbstoffe, insbesondere um sogenannte direktziehende Farbstoffe oder auch um unlösliche anorganische oder organische Pigmente handeln.

Die Gesamtmenge an Farbstoffen beträgt in dem erfindungsgemäßen Mittel etwa 0,01 bis 7 Gew.%, vorzugsweise etwa 0,2 bis 4 Gew.%. Geeignete direktziehende Farbstoffe sind z.B. Triphenylmethanfarbstoffe, aromatische Nitrofarbstoffe, Azofarbstoffe, Chinonfarbstoffe, kationische oder anionische Farbstoffe. Geeignet sind:
Nitrofarbstoffe (blau):
   1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)-amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)amino]-2-nitrobenzol (HC Blue No. 10), 1-[(2,3-Dihydroxypropyl)-amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-(2-Aminoethylamino)-4-[di(2-hydroxyethyl)amino]-2-nitrobenzol, 4-(Di(2-hydroxyethyl)amino)-2-nitro-1-phenylamino-benzol.
Nitrofarbstoffe (rot):
   1-Amino-4-[(2-hydroxyethyl)amino]-2-nitrobenzol (HC Red No. 7), 2-Amino-4,6-dinitro-phenol, 1,4-Diamino-2-nitrobenzol (CI76070), 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-((2-Hydroxyethyl)methylamino)-1-(methylamino)-2-nitrobenzol, 1-Amino-4-((2,3-dihydroxypropyl)amino)-5-methyl-2-nitrobenzol, 1-Amino-4-(methylamino)-2-nitrobenzol, 4-Amino-2-nitro-1-((prop-2-en-1-yl)-amino)-benzol, 4-Amino-3-nitrophenol, 4-[(2-Hydroxyethyl)-amino]-3-nitrophenol, 4-[(2-Nitrophenyl)amino]phenol (HC Orange No. 1), 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 6-Amino-3-((2-hydroxyethyl)amino)-2-nitropyridin, 3-Amino-6-((2-hydroxyethyl)amino)-2-nitropyridin, 3-Amino-6-(ethylamino)-2-nitropyridin, 3-((2-Hydroxyethyl)amino)-6-(methylamino)-2-nitropyridin, 3-Amino-6-(methylamino)-2-nitropyridin, 6-(Ethylamino)-3-((2-hydroxyethyl)amino)-2-nitropyridin, 1,2,3,4-Tetrahydro-6-nitrochinoxalin, 7-Amino-3,4-dihydro-6-nitro-2H-1,4-benzoxazin (HC Red No. 14).
Nitrofarbstoffe (gelb):
   1,2-Diamino-4-nitrobenzol (CI76020), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-(Di(2-hydroxyethyl)amino)-5-nitrophenol, 2-[(2-Hydroxyethyl)amino]-1-methoxy-5-nitrobenzol, 2-Amino-3-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)-amino]-3-nitro-1-trifluormethyl-benzol (HC Yellow No. 6), 1-Chlor-2,4-bis[(2-hydroxyethyl)amino]-5-nitrobenzol (HC Yellow No. 10), 1-Amino-4-((2-aminoethyl)amino)-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)-amino]-3-nitro-benzonitril (HC Yellow No. 14), 4-[(2-Hydroxyethyl)amino]-3-nitro-benzamid (HC Yellow No. 15) 3-((2-Hydroxyethyl)amino)-4-methyl-1-nitrobenzol, 4-Chlor-3-((2-hydroxyethyl)amino)-1-nitrobenzol.
Chinonfarbstoffe:
   1,4-Di[(2,3-dihydroxypropyl)amino]-9,10-anthrachinon, 1,4-Di[(2-hydroxyethyl)amino]-9,10-anthrachinon (CI61545, Disperse Blue 23), 1-[(2-Hydroxyethyl)amino]-4-methylamino-9,10-anthrachinon (CI61505, Disperse Blue No. 3), 2-[(2-Aminoethyl)amino]-9,10-anthrachinon (HC Orange No. 5), 1-Amino-4-hydroxy-9,10-anthrachinon (CI60710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (CI75470, Natural Red 4), 1-[(3-Aminopropyl)amino]-4-methylamino-9, 10-anthrachinon (HC Blue No. 8), 1-[(3-Aminopropyl)-amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 1,4-Diamino-9,10-anthrachinon (CI61100, Disperse Violet No. 1), 1-Amino-4-(methylamino)-9,10-anthrachinon (CI61105, Disperse Violet No. 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-(6-((3-Chlor-4-(methylamino)phenyl)imino)-4-methyl-3-oxo-1,4-cyclohexadien-1-yl)harnstoff (HC Red No. 9), 2-((4-(Di(2-hydroxyethyl)amino)phenyl)amino)-5-((2-hydroxyethyl)amino)-2,5-cyclohexadien-1,4-dion (HC Green No. 1), 5-Hydroxy-1,4-naphthochinon (CI75500, Natural Brown No. 7), 2-Hydroxy-1,4-naphthochinon (CI75480, Natural Orange No. 6), 1,2-Dihydro-2-(1,3-dihydro-3-oxo-2H-indol-2-yliden)-3H-indol-3-on (CI73000), 4-((5-((2-Hydroxyethyl)amino-1-methyl-1H-pyrazol-4-yl)imino)-4,5-dihydro-5-((2-hydroxyethyl)-imino)-1-methyl-1H-Pyrazol-sulfat(1:1),hydrat(1:1).
Basische Farbstoffe:
   9-(Dimethylamino)-benzo[a]phenoxazin-7-ium-chlorid (CI51175; Basic Blue No. 6), Di[4-(diethylamino)phenyl][4-(ethylamino)naphthyl]carbenium-chlorid (CI42595; Basic Blue No. 7), Di-(4-(dimethylamino)phenyl)-(4-(methyl-phenylamino)-naphthalin-1-yl)carbenium-chlorid (CI42563; Basic Blue No. 8), 3,7-Di(dimethylamino)phenothiazin-5-ium-chlorid (CI52015 Basic Blue No. 9), Di[4-(dimethylamino)phenyl][4-(phenylamino)naphthyl]carbenium-chlorid (CI44045; Basic Blue No. 26), 2-[(4-(Ethyl(2-hydroxyethyl)amino)phenyl)azo]-6-methoxy-3-methyl-benzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)phenyl]-[4-(methylamino)phenyl]carbenium-chlorid (CI42535; Basic Violet No. 1), Tri(4-amino-3-methylphenyl)carbenium-chlorid (CI42520; Basic Violet No. 2), Tris[4-(dimethylamino)-phenyl]carbenium-chlorid (CI42555; Basic Violet No. 3), 2-[3,6-(Diethylamino)dibenzopyranium-9-yl]-benzoesäurechlorid (CI45170; Basic Violet No. 10), Di(4-aminophenyl)(4-amino-3-methylphenyl)carbeniumchlorid (CI42510 Basic Violet No. 14), 1,3-Bis[(2,4-diamino-5-methylphenyl)azo]-3-methylbenzol (CI21010; Basic Brown No. 4), 1-[(4-Aminophenyl)azo]-7-(trimethylammonio)-2-naphthol-chlorid (CI12250; Basic Brown No. 16), 3-[(4-Amino-2,5-dimethoxyphenyl)azo]-N,N,N-trimethylbenzolaminiumchlorid (CI112605, Basic Orange No. 69), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)azo]-pyrazol-5-on-chlorid (CI12719; Basic Yellow No. 57), Di[4-(dimethylamino)phenyl]iminomethan-hydrochlorid (CI41000; Basic Yellow No. 2), Bis[4-(diethylamino)phenyl]phenylcarbenium-hydrogensulfat (1:1) (CI42040; Basic Green No. 1), Di(4-(dimethylamino)phenyl)-phenylmethanol (CI42000; Basic Green No. 4), 1-(2-Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid.
Neutrale Azofarbstoffe:
   1- [Di(2-hydroxyethyl)amino]-3-methyl-4-[(4-nitrophenyl) azo]-benzol (CI11210, Disperse Red No. 17), 1-[Di(2-hydroxyethyl)amino]-4-[(4-nitrophenyl)azo]-benzol (Disperse Black No. 9), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin, 2-((4-(Acetylamino)phenyl)azo)-4-methylphenol (CI11855; Disperse Yellow No. 3).
Saure Farbstoffe:
   6-Hydroxy-5-[(4-sulfophenyl)azo]-2-naphthalinsulfonsäuredinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Mono- und Disulfonsäure) (C147005;D&C Yellow No. 10; Food Yellow No. 13; Acid Yellow No. 3), 5-Hydroxy-1-(4-sulfophenyl)-4-[(4-sulfophenyl)azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 4-((4-Amino-3-sulfophenyl)azo)benzolsulfonsäure-dinatriumsalz (CI13015, Acid Yellow No. 9), 5-[(2,4-Dinitrophenyl)amino]-2-phenylaminobenzolsulfonsäure-natriumsalz (CI10385; Acid Orange No. 3), 4-[(2,4-Dihydroxyphenyl)azo]-benzolsulfonsäure Mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]-phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäure-dinatriumsalz (CI14720; Acid Red No. 14), 4-Hydroxy-3-[(2-methoxyphenyl)azo]-1-naphthalinsulfonsäure-mononatriumsalz (CI14710; Acid Red No. 4), 6-Hydroxy-5-[(4-sulfonaphth-1-yl)azo]-2,4-naphthalin-disulfonsäuretrinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäure-dinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430; Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)phenyl)azo]-7-naphthol-1,3-disulfonsäure-dinatriumsalz (CI27290; Acid Red No. 73), 2',4',5',7'-Tetrabrom-3',6'-dihydroxyspiro[isobenzofuran-1(3H),9'-[9H]xanthen]-3-on-dinatriumsalz (CI45380 Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro[isobenzofuran-1(3H),9'(9H)-xanthen]-3-on-dinatriumsalz (CI45425; Acid Red No. 95), 2-Hydroxy-3-((2-hydroxynaphth-1-yl)azo)-5-nitrobenzolsulfonsäure-mononatriumsalz (CI15685; Acid Red No. 184), (2-Sulfophenyl)di[4-(ethyl((4-sulfophenyl)methyl)amino)phenyl]-carbenium-dinatriumsalz Betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxynaphth-1-yl)carbenium-inneres Salz Mononatriumsalz (CI44090; Food Green No. 4; Acid Green No. 50), Bis[4-(diethylamino)-phenyl](2,4-disulfophenyl)carbenium-inneres salz, Natriumsalz (2:1) (CI42045; Food Blue No. 3; Acid Blue No. 1), Bis[4-(diethylamino)phenyl](5-hydroxy-2,4-disulfophenyl)-carbenium-inneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 1-Amino-4-(phenylamino)-9,10-anthrachinon-2-sulfonsäure (CI62055; Acid Blue No. 25), 2-(1,3-Dihydro-3-oxo-5-sulfo-2H-indol-2-yliden)-2,3-dihydro-3-oxo-1H-indol-5-sulfonsäuredinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)-azo]-3-(phenylazo)-2,7-naphthalin-disulfonsäure Dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1), 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red No. 195).

Weitere zur Haarfärbung bekannte und übliche Farbstoffe, die in dem erfindungsgemäßen Färbemittel enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben.

Geeignete haarfärbende Pigmente sind im Anwendungsmedium praktisch unlösliche Farbmittel und können anorganisch oder organisch sein. Auch anorganisch-organische Mischpigmente sind möglich. Bei den Pigmenten handelt es sich vorzugsweise nicht um Nanopigmente. Die bevorzugte Teilchengröße beträgt 1 bis 200 µm, insbesondere 3 bis 150 µm, besonders bevorzugt 10 bis 100 µm. Bevorzugt sind anorganische Pigmente. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal). Besonders bevorzugt sind Pigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt ist. Derartige Pigmente werden beispielsweise unter den Handelsbezeichnung Rona®, Colorona®, Dichrona® und Timiron® von der Firma Merck, Deutschland vertrieben. Organische Pigmente sind z.B. die natürlichen Pigmente Sepia, Gummigutt, Knochenkohle, Kasseler Braun, Indigo, Chlorophyll und andere Pflanzenpigmente. Synthetische organische Pigmente sind z.B. Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrolpigmente.

Das erfindungsgemäße Produkt kann darüber hinaus die für Haarbehandlungsmittel üblichen Zusatzbestandteile enthalten, z.B. Parfümöle in einer Menge von 0,01 bis 0,5 Gew.%; Konservierungsmittel wie z.B. Parabene in einer Menge von 0,01 bis 1,0 Gew.%; Puffersubstanzen, wie z.B. Natriumcitrat oder Natriumphosphat, in einer Menge von 0,1 bis 1,0 Gew.%; Pflegestoffe, wie z.B. Pflanzen- und Kräuterextrakte, Protein- und Seidenhydrolysate, Lanolinderivate, in einer Menge von 0,1 bis 5 Gew.%; physiologisch verträgliche Silikonderivate, wie z.B flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, Glanzgeber und Kämmbarkeitsverbesserer in einer Menge von je etwa 0,01 bis 2 Gew.%.

Das erfindungsgemäße Aerosolschaumprodukt wird in einer druckfesten Aerosolverpackung abgefüllt, welche mit einer Vorrichtung zum Verschäumen (Aersolschaumkopf) versehen wird. Beim Ausbringen aus diesr Aerosolverpackung bildet sich ein Schaum, welcher leicht in das Haar eingearbeitet werden kann und beim Einarbeiten auf dem Haar schnell zusammenbricht.

Das erfindungsgemäße kosmetische Mittel wird angewendet, indem es in einer zur Erzielung des gewünschten haarpflegenden oder haarfestigenden Effektes ausreichenden Menge auf feuchtes, handtuchtrockenes Haar aufgetragen und eingearbeitet wird und ohne Ausspülen im Haar belassen wird. Anschließend kann die Frisur in üblicher Weise geformt werden bzw. können die Haare eingelegt und zum Schluß trocken gefönt werden. Es ist aber auch möglich, das Mittel direkt auf trockenem Haar anzuwenden.

Es wird ein Schaum mit guter, kompakter Schaumqualität erhalten, der auf dem Haar schnell zusammenbricht und gut in das Haar einarbeitbar ist. Er zeichnet sich aus durch gute Föneigenschaften, einen angenehmen, glatten Griff des feuchten und des trockenen Haares, gute Elastizität, keine Belastung, einen schönen Glanz und starke Festigung.

Die nachfolgenden Beispiele sollen den Gegenstand der Erfindung näher erläutern.

### Beispiele

| **Beispiel 1A:** Erfindungsgemäßer Aerosol-Haarschaum: | |
|---|---|
| 1 g | Chitosan |
| 1 g | Celquat® L200 (Copolymer aus Hydroxyethylcellulose und Diallyldimethylammoniumchlorid; Polyquaternium-4) |
| 0,3 g | Ameisensäure (85%ig) |
| 0,2 g | Laureth-4 |
| 0,2 g | Parfüm |
| 0,15 g | Cetyltrimethylammoniumchlorid |
| 10 g | Ethanol |
| ad 100 g | Wasser |

| **Beispiel 1B:** Vergleichsrezeptur | |
|---|---|
| 0,67 g | Chitosan |
| 1,33 g | Celquat® L200 (Copolymer aus Hydroxyethylcellulose und Diallyldimethylammoniumchlorid; Polyquaternium-4) |
| 0,22 g | Ameisensäure (85%ig) |
| 0,2 g | Laureth-4 |
| 0,2 g | Parfüm |
| 0,15 g | Cetyltrimethylammoniumchlorid |
| 10 g | Ethanol |
| ad 100 g | Wasser |

Die Wirkstoffmischungen wurden jeweils im Verhältnis 92:8 mit Propan/Butan 4.8 bar als Treibmittel in einer Aerosoldose mit Schaumventil abgefüllt. In Salonversuchen wurden Testpersonen mit jeweils gleichen Schaummengen behandelt. Die behandelten Haare wurden durch ausgebildete Friseurfachkräfte sensorisch beurteilt. Dabei wurden die folgenden Beurteilungen erhalten:
Schaum 1A:
   Der Schaum hat eine schöne, kompakte Schaumqualität und läßt sich gut im Haar verteilen. Die Föneigenschaften sind sehr gut. Das Haar fühlt sich nach dem Trocknen sehr gut an, hat einen glatten Griff, gute Elastizität, keine Belastung, einen schönen Glanz und eine starke Festigung.
Schaum 1B:
   Der Schaum hat eine gute Schaumqualität und läßt sich gut im Haar verteilen. Nach dem Auftragen schäumt der Schaum im Haar sehr stark auf. Das Haar fühlt sich unangenehm, sehr glitschig und seifig an. Die Föneigenschaften sind nicht positiv, das Gleitvermögen ist stark erschwert. Nach dem Trocknen sieht das Haar stumpf aus und hat außer einer starken Festigung nicht die besonderen positiven Eigenschaften wie nach Behandlung mit Schaum 1A.

| **Beispiel 2:** Pumpschaum | |
|---|---|
| 0,5 g | Chitosan |
| 0,5 g | Celquat® L200 (Copolymer aus Hydroxyethylcellulose und Diallyldimethylammoniumchlorid; Polyquaternium-4) |
| 1 g | Abilquat® 3272 (Quaternium-80, 50%ig in Propylenglykol) |
| 0,5 g | Cetyltrimethylammoniumchlorid |
| 0,15 g | Ameisensäure (85%ig) |
| 5 g | Ethanol |
| ad 100 g | Wasser |

| **Beispiel 3:** Aerosol-Schaumfestiger | |
|---|---|
| 1 g | Chitosan |
| 1 g | Celquat® L200 (Copolymer aus Hydroxyethylcellulose und Diallyldimethylammoniumchlorid; Polyquaternium-4) |
| 0,85 g | Pyrrolidoncarbonsäure |
| 1,5 g | Vinylpyrrolidon/Vinylacetat Copolymer (Luviskol® VA64) |
| 0,2 g | Laureth-4 |
| 0,3 g | Parfüm |
| 0,2 g | Cetyltrimethylammoniumchlorid |
| ad 100 g | Wasser |

Die Wirkstoffmischung wird im Verhältnis 92:8 mit Propan/Butan 4.8 bar als Treibmittel in einer Aerosoldose mit Schaumventil abgefüllt.

| **Beispiel 4:** Aerosol-Schaumfestiger | |
|---|---|
| 1,25 g | Chitosan (MW 20.000-100.000) |
| 0,3 g | Chitosan (MW 300.000-700.000) |
| 1,25 g | Celquat® L200 (Copolymer aus Hydroxyethylcellulose und Diallyldimethylammoniumchlorid; Polyquaternium-4) |
| 0,4 g | Ameisensäure (85%ig) |
| 0,25 g | Parfüm |
| 0,2 g | Cetyltrimethylammoniumchlorid |
| 10 g | Ethanol |
| ad 100 g | Wasser |

Die Wirkstoffmischung wird im Verhältnis 92:8 mit Propan/Butan 4.8 bar als Treibmittel in einer Aerosoldose mit Schaumventil abgefüllt.

| **Beispiel 5:** Farb-Schaumfestiger | |
|---|---|
| 1 g | Chitosan |
| 1 g | Celquat® L200 (Copolymer aus Hydroxyethylcellulose und Diallyldimethylammoniumchlorid; Polyquaternium-4) |
| 0,3 g | Ameisensäure (85%ig) |
| 0,2 g | Laureth-4 |
| 0,2 g | Parfüm |
| 0,11 g | 3-(((2-Nitro-4-(trifluormethyl)phenyl)amino)-1,2-propandiol |
| 0,15 g | Cetyltrimethylammoniumchlorid |
| 10 g | Ethanol |
| ad 100 g | Wasser |

Die Wirkstoffmischung wird im Verhältnis 94:6 mit Propan/Butan 5 bar als Treibmittel in einer Aerosoldose mit Schaumventil abgefüllt.

| **Beispiel 6:** Farbmousse rot | |
|---|---|
| 5,00 g | Timiron® starlight red (Merck) ¹⁾ |
| 1 g | Chitosan |
| 1 g | Celquat® L200 (Copolymer aus Hydroxyethylcellulose und Diallyldimethylammoniumchlorid; Polyquaternium-4) |
| 0,3 g | Ameisensäure (85%ig) |
| 0,2 g | Laureth-4 |
| 0,2 g | Parfüm |
| 0,15 g | Cetyltrimethylammoniumchlorid |
| 10 g | Ethanol |
| ad 100 g | Wasser |

| | |
|---|---|
| ¹⁾ Mica/Titandioxid-Pigment mit roter Reflektionsfarbe | |

Die Wirkstoffmischung wird im Verhältnis 94:6 mit Propan/Butan 5 bar als Treibmittel in einer Aerosoldose mit Schaumventil abgefüllt.

## Patentansprüche

1. Aerosolschaum- oder Pumpschaumprodukt zur Haarbehandlung, bestehend aus einer verschäumbaren Zusammensetzung mit einem Gehalt an
(A) mindestens einem kationischen Cellulosederivat das ausgewählt ist aus Copolymeren aus Hydroxyethylcellulose und Diallyldimethylammoniumchlorid (Polyquaternium-4),
(B) Chitosan,
(C) mindestens einer Säure zur Neutralisation des Chitosans, wobei die Säure ausgewählt ist aus Ameisensäure, Weinsäure, Äpfelsäure, Zitronensäure, Pyrrolidoncarbonsäure und Salzsäure und
(D) einem geeigneten Lösungsmittelsystem,
wobei das Gewichtsverhältnis des kationischen Cellulosederivats (A) zum Chitosan (B) kleiner als 2 ist und die Zusammensetzung entweder zusammen mit mindestens einem Treibmittel (E) in einer druckfesten Verpackung oder ohne Treibmittel in einer Verpackung mit einer mechanischen Vorrichtung zum Verschäumen abgefüllt ist , sowie gegebenenfalls mindestens einem Tensid (F), mindestens einem haarfärbenden Stoft,
Parfümölen in einer Menge von 0,01 bis 0,5 Gew.%; Konservlerungsmitten in einer Menge von 0,01 bis 1,0 Gew.%; Pufferstanzen, in einer Menge von 0,1 bis 1,0 Gew.%; Pflegastoffen in einer Menge von 0,1 bis 5 Gew.%; physiologische verträglichen Silikonderivaten in einer Menge von 0,05 bis 20 Gew.%; Lichtschutzmitteln Antioxidantien, Radikalfängern Antischuppenwirkstoffen Glanzgebern und Kämmbarkeitsverbesseren in einer Menge von je 0,01 bis 2 Gew.%, sowie gegebenenfalls mindestens einem nichtionischen film bildenden Polymeren.

2. Schaumprodukt nach Anspruch 1, **dadurch gekennzeichnet, dass** das Cellulosederivat (A) ausgewählt ist aus Copolymeren aus Hydroxyethylcellulose und Diallyldimethylammoniumchlorid (Polyquaternium-4) mit einem Molekulargewicht zwischen zwischen 200.000 und 400.000 und einem Stickstoffgehalt von 1,5 bis 3%.

3. Schaumprodukt nach einem der vorhergehenden Ansprüche, **dadurch gekennzeichnet, dass** das Chitosan ein Molekulargewicht über 100.000 und einen Deacetylierungsgrad von mindestens 60% aufweist.

4. Schaumprodukt nach Anspruch 1, 2 oder 3, **dadurch gekennzeichnet, dass** das Tensid (F) ausgewählt ist aus
- nichtionischen Tensiden, ausgewählt aus Fettsäureglyceridethoxylaten, Fettalkoholethoxylaten, Fettaminethoxylaten, Fettsäurealkanolamidethoxylaten, Fettsäureesterethoxylaten, ethoxylierten Fettsäureauckerestern, ethoxylierten Sorbitanfettsäureestern, nichtethoxylierten Fettsäurezuckerestern und Alkylpolyglycosiden und
- kationischen Tensiden der allgemeinen Formel (I)
N⁽⁺⁾R¹R²R³R⁴ X⁽⁻⁾ (I)
wobei R1 bis R4 unabhängig voneinander Alkylgruppen, Arylgruppen, Alkoxygruppen, Polyoxyalkylengruppen, Alkylamidogruppen, Hydroxyalkylgruppen oder Alkarylgruppen mit 1 bis 22 C-Atomen bedeuten und X⁽⁻⁾, ein Anion darstellt.

5. Aerosolschaumprodukt nach einem der vorstehenden Anspruche, **dadurch gekennzeichnet, dass** das Treibmittel (E) ausgewählt ist aus Propan, i-Butan, n-Butan oder Dimethylether oder aus Mischungen dieser Treibgase.

6. Schaumprodukt nach einem der vorstehenden Ansprüche, **dadurch gekennzeichnet, dass** das Cellulosederivat (A) in einer Menge von 0,1 bis 10 Gew.%, das Chitosan (B) in einer Menge von 0,1 bis 10 Gew.%, die Säure (C) in einer Menge, die ausreichend ist, um das Chitosan zu 50 bis 100% zu neutralisieren, das Treibmittel (E) in einer Menge von 1 bis 20 Gew.%, das Lösungsmittelsystem (D) in einer Menge von 50 bis 98 Gew.% und das Tensid (F) in -einer Menge von 0 bis 15 Gew.% enthalten ist.

7. Schaumprodukt nach einem der vorstehenden Ansprüche **dadurch gekennzeichnet, dass** das nichtionische Polymer ausgewählt ist aus Polyvinylpyrrolidon und Copolymeren von Vinylpyrrolidon und ethylenisch ungesättigten nicht ionischen Comonomeren.

8. Schaumprodukt nach einem der vorstehenden Ansprüche, **dadurch gekennzeichnet, dass** es einen Gehalt aufweist an
(A) 0,5 bis 5 Gew.% eines Copolymeren aus Hydroxyethylcellulose und Diallyldimethylammoniumchlorid (Polyquaternium-4)
(B) 0,5 bis 5 Gew.% Chitosan eines Molekulargewichts von 200.000 bis 700.000 und eines Deacetylierungsgrades von mindestens 60%,
(C) einer Säure in einer Menge, die ausreichend ist, um das Chitosan zu 50 bis 100% zu neutralisieren,wobei die Säure ausgewählt ist aus Ameisensäure, Weinsäure, Äpfelsäure, Zitronensäure, Pyrrolidoncarbonsäure und Salzsäure,
(D) 50-98 Gew.% eines Lösungsmittels, ausgewählt aus Wasser, C2-3-Alkoholen oder deren Gemisch,
(E) 1 bis 20 Gew.% eines Treibmittels ausgewählt aus C3- und C4-Kohlenwasserstoffen,
wobei das Gewichtsverhältnis des kationischen Cellulosederivats (A) zum Chitosan (B) von 1:2 bis kleiner als 2:1 ist.

## Claims

1. An aerosol mousse or pump mousse product for hair treatment consisting of a foamable composition having a content of
(A) at least one cationic cellulose derivative selected from copolymers of hydroxyethylcellulose and diallyldimethylammonium chloride (Polyquaternium-4),
(B) chitosan,
(C) at least one acid for neutralization of said chitosan, the acid being selected from formic acid, tartaric acid, malic acid, citric acid, pyrrolidone carboxylic acid and hydrochloric acid, and
(D) a suitable solvent system,
wherein the weight ratio of the cationic cellulose derivative (A) to the chitosan (B) is smaller than 2 and the composition is filled either together with at least one foaming agent (E) into a pressure-resistant packaging, or without a foaming agent into a packaging having a mechanical device for producing foam, and optionally at least one surfactant (F), at least one hair-coloring agent,
perfume oils in an amount of 0.01 to 0.5 percent by weight; preservatives in an amount of 0.01 to 1.0 percent by weight; buffering agents in an amount of 0.1 to 1.0 percent by weight; conditioning agents in an amount of 0.1 to 5 percent by weight; physiologically compatible silicone derivatives in an amount of 0.05 to 20 percent by weight; photoprotective agents, antioxidants, free radical scavengers, antidandruff agents, gloss agents and detangling agents, each in an amount of 0.01 to 2 percent by weight, and optionally at least one nonionic film-forming polymer.

2. The mousse product according to Claim 1, **characterized in that** the cellulose derivative (A) is selected from copolymers of hydroxyethylcellulose and diallyldimethylammonium chloride (Polyquaternium-4) having a molecular weight between 200,000 and 400,000 and a nitrogen content of 1.5 to 3%.

3. The mousse product according to either one of the preceding claims, **characterized in that** the chitosan has a molecular weight of greater than 100,000 and a deacetylation degree of at least 60%.

4. The mousse product according to Claim 1, 2 or 3, **characterized in that** the surfactant (F) is selected from
- nonionic surfactants, selected from fatty acid glyceride ethoxylates, fatty alcohol ethoxylates, fatty amine ethoxylates, fatty acid alkanolamide ethoxylates, fatty acid ester ethoxylates, ethoxylated fatty acid sucrose esters, ethoxylated sorbitan fatty acid esters, non-ethoxylated fatty acid sucrose esters and alkyl polyglycosides, and
- cationic surfactants corresponding to the following general formula (I)
N⁽⁺⁾R¹R²R³R⁴X⁽⁻⁾ (I)
wherein each of R1 to R4 is independently an alkyl group, aryl group, alkoxy group, polyoxyalkylene group, alkylamido group, hydroxyalkyl group, or alkaryl group, having from 1 to 22 carbon atoms, and X⁽⁻⁾, represents an anion.

5. The aerosol mousse product according to any one of the preceding claims, **characterized in that** the foaming agent (E) is selected from propane, i-butane, n-butane, dimethyl ether, or mixtures of said propellant gases.

6. The mousse product according to any one of the preceding claims, **characterized in that** the mousse product contains the cellulose derivative (A) in an amount of 0.1 to 10% by weight, the chitosan (B) in an amount of 0.1 to 10% by weight, the acid (C) in an amount sufficient to neutralize 50 to 100% of the chitosan, the propellant (E) in an amount of 1 to 20% by weight, the solvent system (D) in an amount of 50 to 98% by weight, and the surfactant (F) in an amount of 0 to 15% by weight.

7. The mousse product according to any one of the preceding claims, **characterized in that** the nonionic polymer is selected from polyvinylpyrrolidone and copolymers of vinylpyrrolidone and ethylenically unsaturated nonionic comonomers.

8. The mousse product according to any one of the preceding claims, **characterized in that** the mousse product has a content of
(A) 0.5 to 5 percent by weight of a copolymer of hdroxyethylcellulose and diallyldimethylammonium chloride (Polyquaternium-4)
(B) 0.5 to 5 percent by weight chitosan of a molecular weight of 200,000 to 700,000 and a deacetylation degree of at least 60%,
(C) an acid in an amount sufficient to neutralize 50 to 100% of the chitosan, said acid being selected from formic acid, tartaric acid, malic acid, citric acid, pyrrolidone carboxylic acid and hydrochloric acid,
(D) 50-98% by weight of a solvent, selected from water, C2-3 alcohols or a mixture thereof; and;
(E) 1 to 20% by weight of a foaming agent selected from C3 and C4 hydrocarbons,
wherein the weight ratio of the cationic cellulose derivative (A) to the chitosan (B) is from 1:2 to smaller than 2:1.

## Revendications

1. Produit sous forme de mousse aérosol ou de mousse en flacon-pompe pour traitement capillaire comprenant une composition apte au moussage présentant une teneur en
(A) au moins un dérivé de cellulose cationique qui est choisi parmi des copolymères d'hydroxyéthylcellulose et de chlorure de diméthyldiallylammonium (polyquatemium-4),
(B) chitosane,
(C) au moins un acide de neutralisation du chitosane, l'acide étant choisi parmi l'acide formique, l'acide tartrique, l'acide malique, l'acide citrique, l'acide pyrrolidonecarboxylique et l'acide chlorhydirique et
(D) un système de solvant adapté,
le rapport de poids du dérivé de cellulose cationique (A) par rapport au chitosane (B) étant inférieur à 2 et la composition étant remplie soit avec au moins un agent moussant (E) dans un conditionnement résistant à la pression, soit sans agent moussant dans un conditionnement doté d'un dispositif mécanique de moussage, ainsi qu'éventuellement au moins un agent tensioactif (F), au moins une substance colorant les cheveux,
des huiles parfumées dans une quantité de 0,01 à 0,5 % en poids ; des agents conservateurs dans une quantité de 0,01 à 1,0 % en poids ; des sels tampons dans une quantité de 0,1 à 1,0 % en poids ; des substances d'entretien dans une quantité de 0,1 à 5 % en poids ; des dérivés de silicone physiologiquement compatibles dans une quantité de 0,05 à 20 % en poids ; des inhibiteurs de lumière, des antioxydants, des capteurs de radicaux libres, des principes actifs antipelliculaires, des brillanteurs et des améliorateurs de peignabilité dans une quantité de 0,01 à 2 % en poids chacun, ainsi qu'éventuellement au moins un polymère filmogène non ionique.

2. Produit sous forme de mousse selon la revendication 1, **caractérisé en ce que** le dérivé de cellulose (A) est choisi parmi des copolymères d'hydroxyéthylcellulose et de chlorure de diméthyldiallylammonium (polyquatemium-4) présentant un poids moléculaire situé entre 200.000 et 400.000 et une teneur en azote de 1,5 à 3%.

3. Produit sous forme de mousse selon l'une des revendications précédentes, **caractérisé en ce que** le chitosane présente un poids moléculaire supérieur à 100.000 et un degré de déacétylation d'au moins 60 %.

4. Produit sous forme de mousse selon la revendication 1, 2 ou 3, **caractérisé en ce que** l'agent tensioactif (F) est choisi parmi
- des agents tensioactifs non ioniques, choisis parmi les éthoxylates de glycéride d'acides gras, les éthoxylates d'alcool gras, les éthoxylates d'amine grasse, les étholxylates d'alkanolamides d'acides gras, les éthoxylates d'esters d'acides gras, les sucroesters d'acides gras ethoxylés, les esters d'acides gras de sorbitan, les sucroesters d'acides gras non ethoxylés et les alkylpolyglycosides et
- des agents tensioactifs cationiques de la formule générale (I)
N⁽⁺⁾R¹R²R³R⁴X⁽⁻⁾ (I)
où R1 à R4 représentent des groupes alkyles, des groupes aryles, des groupes alkoxy, des groupes polyoxyalkyles, des groupes alkylamido, des groupes hydroxyalkyles ou des groupes alkyles avec 1 à 22 atomes C et X⁽⁻⁾ représente un anion.

5. Produit sous forme de mousse selon l'une des revendications précédentes, **caractérisé en ce que** l'agent moussant (E) est choisi parmi le propane, le i-butane, le n-butane ou l'éther diméthylique ou parmi des mélanges de ces gaz propulseurs.

6. Produit sous forme de mousse selon l'une des revendications précédentes, **caractérisé en ce que** le dérivé de cellulose (A) est compris dans une quantité de 0,1 à 10 % en poids, le chitosane (B) dans une quantité de 0,1 à 10 % en poids, l'acide (C) dans une quantité qui est suffisante pour neutraliser le chitosane à 50 à 100 %, l'agent moussant (E) dans une quantité de 1 à 20 % en poids, le système de solvant (D) dans une quantité de 50 à 98 % en poids et l'agent tensioactif (F) dans une quantité de 0 à 15 % en poids.

7. Produit sous forme de mousse selon l'une des revendications précédentes, **caractérisé en ce que** le polymère non ionique est choisi parmi le polyvinylpyrrolidone et des copolymères de vinylpyrrolidone et des comonomères non ioniques éthyléniquement insaturés.

8. Produit sous forme de mousse selon l'une des revendications précédentes, **caractérisé en ce qu'**il présente une teneur en
(A) 0,5 à 5 % en poids d'un copolymère d'hydroxyéthylcellulose et de chlorure de diméthyldiallylammonium (polyquatemium-4)
(B) 0,5 à 5 % en poids de chitosane d'un poids moléculaire de 200.000 à 700.000 et d'un degré de déacétylation d'au moins 60 %,
(C) un acide dans une quantité qui est suffisante pour neutraliser le chitosane à 50 à 100%, l'acide étant choisi parmi l'acide formique, l'acide tartrique, l'acide malique, l'acide citrique, l'acide pyrrolidonecarboxylique et l'acide chlorhydirique,
(D) 50 à 98 % en poids d'un solvant, choisi parmi l'eau, les alcools C2-3 ou leur mélange,
(E) 1 à 20 % en poids d'un agent moussant choisi parmi les hydrocarbures C3 et C4,
le rapport de poids du dérivé de cellulose cationique (A) par rapport au chitosane (B) étant compris dans une plage allant de 1:2 à moins de 2:1.
